# EUROPEAN PATENT SPECIFICATION

(11) **EP 2 094 181 B1**
(45) Date of publication and mention of the grant of the patent: **18.07.2012**
(21) Application number: 07864117.2
(22) Date of filing: 08.11.2007
(51) Int. Cl.: A61B 19/08, G06F 1/16

(54) **MONITOR DRAPE WITH VENTS**
MONITORTUCH MIT LUFTLÖCHERN
CHAMP OPÉRATOIRE DE CONTRÔLE POSSÉDANT DES OUVERTURES DE VENTILATION

(30) Priority: 09.11.2006 US 558431
(43) Date of publication of application: 02.09.2009
(73) Proprietor: Abbott Medical Optics Inc., Santa Ana, CA 92705-4933 (US)
(72) Inventor: EDWARDS, Craig, Mission Viejo, CA 92691 (US)
(74) Representative: HOFFMANN EITLE
(86) International application number: PCT/US2007/084093
(87) International publication number: WO 2008/060947

(56) References cited:
- US-A1- 2002 069 882
- US-A1- 2003 153 810
- US-A1- 2006 161 137

## Description

### BACKGROUND OF THE INVENTION

The present invention relates to devices and systems used in surgery. Exemplary embodiments specifically relate to the maintenance of a sterile field in the area around the surgical procedure during treatments and/or measurements of the eye.

Surgical and diagnostic procedures involving the tissues of the eye often employ sophisticated surgical systems or devices. To facilitate accurate measurements and therapies to the tissues of the eye, these system often display information and receive inputs while the system is in use for a particular patient. Flat panel display screens are being used with greater and greater frequency to display information to the surgical personnel, and in many cases, to receive input or commands from the surgeon (or other medical professionals) during medical procedures through the use of flat panel touch screen structures.

More generally, touch panel display screens are often placed in or near the sterile field for use in preparation for, during, and/or after a wide variety of surgical or diagnostic procedures. Since complex display screens and computerized systems may not be easily sterilized, it can be advantageous to place a sterile drape over the display screen. However, existing sterile drapes may not be optimized for use with surgical touch screen displays. For example some existing sterile drapes may hinder the display of precise images and graphic information, and/or may hinder inputting of commands to the touch screen. Other sterile drapes may not be easily and effectively positioned for surgery or readily removed after surgery, and they may not fit a wide range of display screen structures. The difficulty in accurate fitting of sterile drapes to flat panel displays may result, for example, in either bunching of the drape material (when elastic or tape is used to allow a drape to be used with a range of different display sizes) or excessive surgical drape inventory costs and complexity (when a large number of different specialized drapes are needed for a number of different displays).

Accordingly, it would be desirable to provide a sterile drape optimized for use with flat panel display screens and touch screen displays. It would be further desirable to provide a drape having a high transparency and which allows an accurate transmittance of tactile pressure, but which can be easily deployed on to and removed off of a flat panel touch screen, ideally without bunching of the material, without relying on repeated application of adhesives onto the display surface (which might otherwise build-up and decrease display resolution), and without other deleterious effects on the life or functioning of a surgical touch screen display, all while maintaining patient safety and the appropriate sterile field.
US 2003/0153810 A1 provides a surgical drape for covering a viewing screen. It has a cruciform configuration with four arms that are foldable about a viewing screen.
US 2002/0069882 A1 discloses a windowed medical drape for a monitor comprising a polymer bag with clear polymer window inserted therein for placement against the monitor.

### BRIEF SUMMARY OF THE INVENTION

The present invention generally provides improved sterile drapes for flat panel displays, and often for flat panel touch screen displays. Embodiments of the invention may allow a sterile field to be easily established before use, and may maintain the sterile field during use of a touch screen user interface by surgical personnel during surgery. A simple folded sheet of a suitable transparent sterile material is fused along its sides, with a front sheet portion of the material being allowed to hang freely along the display surface. The rear sheet is shorter than the front, and the drape is open at the bottom with ventilation holes along the top. This simple open arrangement may avoid wrinkles or bunching of the material along the display (which might otherwise result from the use of adhesives, tape, or elastic) and allows the user to view and actuate a touch screen user interface while accommodating convective cooling of the flat panel display. After use, the drape is readily lifted off the screen, inhibiting any inadvertent yanking on the display screen or its support structure.

The present invention provides a sterile drape for a flat panel display having a display surface. The sterile drape comprises a sterile front sheet at least a large as the display surface, the front sheet comprising a thin layer of a transparent material and having a top edge and a bottom edge. A rear sheet has at least one opening for ventilation, and the rear sheet is attached to the front sheet near the top edge. A rear bottom edge of the rear sheet is detached from the front sheet so as to form a hood with an open bottom, the front sheet and the rear sheet configured for draping over the flat panel display so as to maintain a sterile field adjacent the display surface.

Typically, the front sheet is substantially rectangular in shape, and a plurality of openings may be distributed across the rear sheet near the top edge. The openings may be disposed along the top and/or rear surface of the flat panel display when the sterile drape is positioned for use. The rear sheet is significantly shorter than a height of the front sheet between the top edge and the bottom edge so as to leave a lower rear portion of the flat panel display uncovered. This can help to accommodate support structures associated with the display without delaying surgical preparations for draping structures that the surgical staff will not touch and/or contaminate during normal surgical system use.

The rear sheet is formed from a folded extension of the front sheet, with the left and right edges of the front and rear sheets being fused together. The front sheet and the rear sheet will typically comprise a thin layer of flexible polymer material suitable for viewing the display surface therethrough, with the layer often being configured to accommodate the transmission of tactile touch-screen inputs from the sterile field to the flat panel display while maintaining the sterile field. The bottom opening between the front sheet and the rear sheet, together with a plurality of openings distributed between left and right edges near the top edge, may allow sufficient air heated by the flat panel display to be ventilated that the entire flat panel display is cooled. A single fold may separate the front and rear sheets along their entire width, although more complex fold and/or seam arrangements may also be included, optionally with the rear sheet having a top portion extending along a top surface of the display. Nonetheless, the rear sheet generally will extend downward and/or rearward from the top edge of the front sheet.

The front sheet and the rear sheet have a bottom opening between them, and the bottom opening being such that the flat panel display can slide into that opening so that the front sheet remains aligned with the display surface when touch commands are transmitted from a user in the sterile field thereto. The bottom remains open when the flat panel display is between the front and rear sheets, and the at least one ventilation opening in the thin transparent material near the top edge allows ventilation to flow in through the bottom opening and then out.

### BRIEF DESCRIPTION OF THE DRAWINGS

Fig. 1 is a schematic illustration of an eye surgery system having a user interface making use of a flat panel display touch screen, in which an embodiment of a surgical drape of the present invention is disposed over the touch screen display surface of the flat panel.

Fig. 2A is a front perspective view of a flat panel touch screen display with an exemplary embodiment of a surgical drape of the present invention disposed thereon.

Fig. 2B is a rear perspective view of the flat panel touch screen display and surgical drape of Fig. 2A.

Fig. 3 is an alternative rear perspective of the surgical drape of Fig. 2A.

Fig. 4 is a rear view of the surgical drape of Fig. 2A, schematically showing how the rear sheet can be formed from an extension of the front sheet material by folding the material along the top edge.

### DETAILED DESCRIPTION OF THE INVENTION

According to embodiments of the present invention, a sterile drape for use during surgical procedures is provided. More specifically, the present invention relates to a sterile drape used to maintain a sterile field along a touch screen, such as those used with a variety of surgical systems and devices. By way of example, the invention can be applied to eye treatment systems such as phacoemulsification system for treatment of tissues within the eye, laser vision correction systems, and the like, generally to maintain a sterile field along flat panel display and/or touch screens.

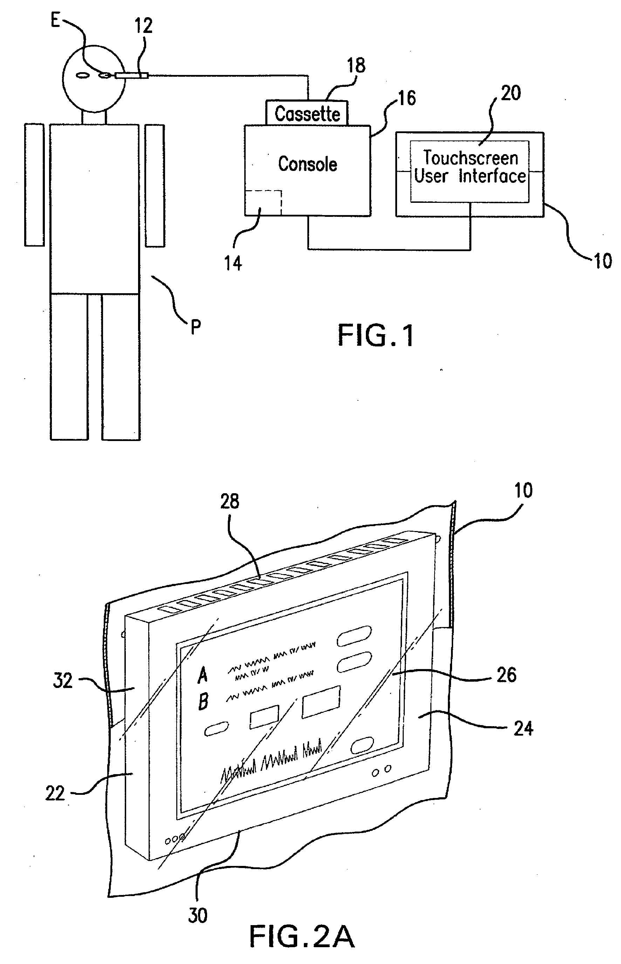

One embodiment of a surgical drape 10 used with an eye surgery system is shown schematically in Figure 1. A probe handpiece 12 is used by a surgeon to treat an eye E of a patient P. The probe here may comprise, for example, a phacoemulsification probe used to treat cataracts of the eye E. A tip of probe 12 is inserted into the eye and energized to transmit ultrasound energy that breaks up tissues, and simultaneous aspiration and irrigation fluid flows are directed through the probe tip to remove the tissues while maintaining the internal eye fluid volume and pressure.

The operation of probe 12 is controlled by a processor 14 of a surgical console per the commands of the surgeon. A cassette 18 couples the probe 12 to the console 16, and the surgeon monitors the status of the surgical system and inputs commands to the surgical system at least in part using a touch screen user interface 20 via a flat panel touch screen display. Surgical drape 10 extends over the touch screen display of the user interface, allowing the surgeon to see the information presented by the display and to transmit commands to processor 14 by touching the display screen with a finger, a tool, and/or the like. Suitable eye surgery systems for use with surgical drape 10 include those commercially available from Advanced Medical Optics, Inc. of Santa Ana, California; Alcon Laboratories of Fort Worth, Texas, Bausch and Lomb of Rochester, New York, and others. A wide range of alternative eye treatment and/or measurement systems and devices might also employ similar surgical drapes, including the laser vision correction systems and/or wavefront measurement systems commercially available from VISX of Santa Clara, California and others. Embodiments of the surgical drapes of the present invention may also find use with a wide variety of alternative medical treatment and/or diagnostic systems used to address disease states of a wide variety of tissues in the body in a range of different clinical and research settings.

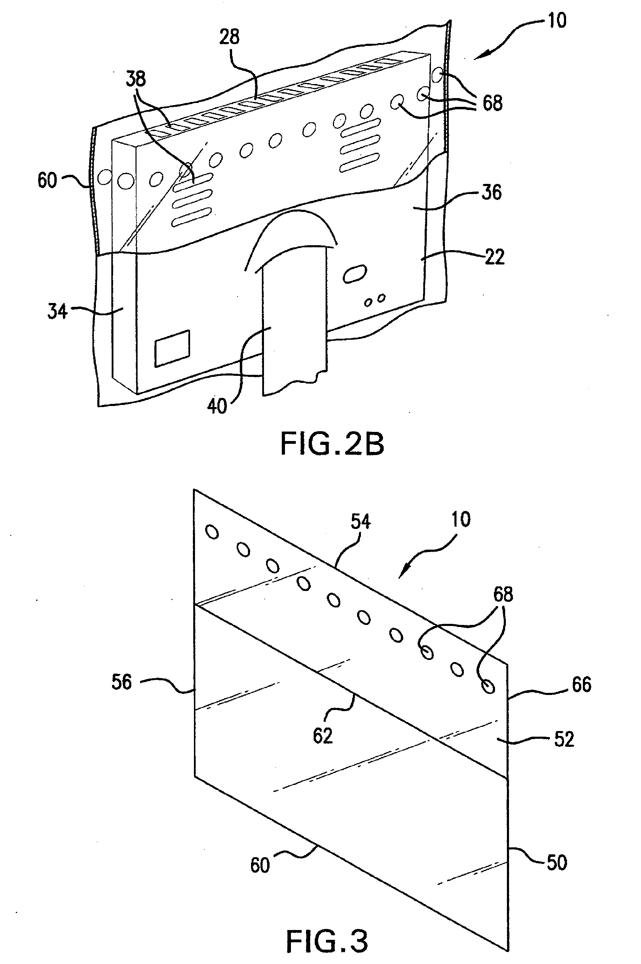

Referring now to Figs. 2A and 2B, surgical drape 10 is seen disposed over a flat panel display 22. Flat panel 22 generally includes a front 24 having a touch screen display surface 26. Flat panel 22 generally includes a top 28 and bottom 30 surfaces, with a height therebetween. Left 32 and right 34 surfaces of flat panel generally define its width, and the height and width of the flat panel are typically greater than a thickness between front 24 and a rear surface 36. Vents 38 may be formed in the top 28, bottom 30, left 32, right, 34, and/or rear 36 surfaces of the flat panel to allow cooling of the display, electronics, and the like, and vents might also be found in front surface 24 outside of display 26 in some embodiments. A support structure 40 extends from a lower portion of rear surface 36. Exemplary touch screen flat panel displays suitable for use in surgical systems include those commercially available from L.G. Phillips located in Korea, and the like.

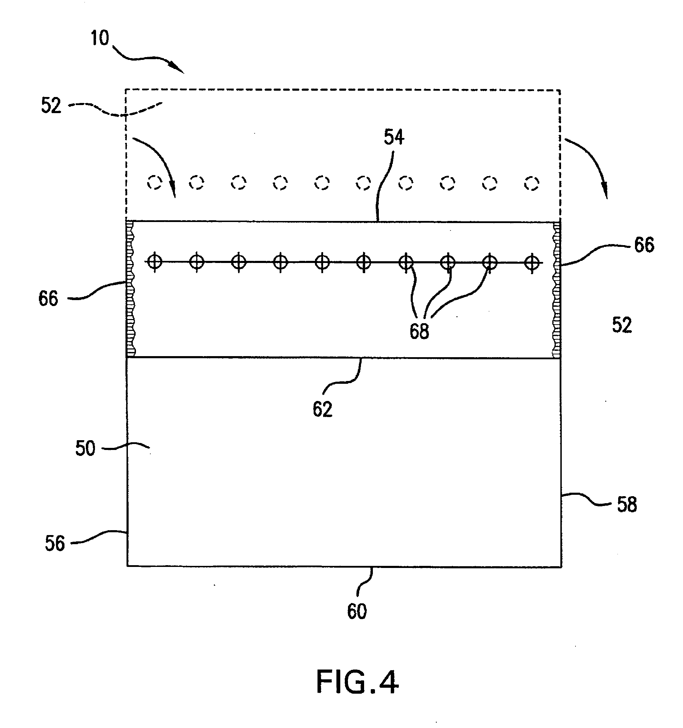

Referring now to Figs. 3 and 4, surgical drape 10 generally comprises a front sheet 50 and a rear sheet 52, both formed of a thin layer of transparent material. The front and rear sheets may be of a generally rectangular shape corresponding to (though often larger and/or taller in height than) the shape of the flat panel display and/or the display screen, although other suitable shapes might also be used. Regardless, surgical drape 10 will include a top edge 54, a right edge 56, and a left edge 58, with the front and rear sheets 50, 52 each having an associated bottom edge 60, 62, respectively. Bottom edge 60 of front sheet 50 will extend below bottom edge 62 to cover display 26 while leaving a lower portion of the rear surface 36 of the flat panel uncovered.

The transparent material from which front sheet 50 and rear sheet 52 are formed generally allows easy viewing of the information displayed on the flat panel touch screen. The thin layer accommodates the transmission of tactile pressure from the sterile field to the touch screen for providing touch commands. The thin layer may also be of sufficient flexibility so that the front sheet substantially conforms to the display surface of the flat panel touch screen. Substantially conforming to the front sheet of the display surface may advantageously reduce optical distortions and ease the operation of the touch screen. The material may be of a suitable polymer, for example, a high clarity, low density polyethylene resin such as that commercially available from Dow Chemical under model number 503A, Certene™ LDF 221 from Muehlstein, Chevron Phillips 5355, and/or the like. Sheets 50 and 52 may have a thickness in a range from 30 µm to 300µm (about 1 mil to about 10 mil), with an exemplary embodiment having a thickness of 50 µm to 100 µm (about 2 mil to about 4 mil). In some embodiments, the rear sheet may be formed of a different and/or even non-transparent material, since the rear sheet does not cover the display portion of the display screen.

As can be understood with reference to Fig. 4, rear sheet 52 is formed by folding an extension of the material of front sheet 50 along top edge 54, so that the top edge of surgical drape comprises a folded edge. Side weld heatseals 66 are formed between the adjacent right edges of front sheet 50 and rear sheet 52, and also between the adjacent left edges. More generally, as can be seen in Figs. 2A an 2B, the rear sheet is attached to the front sheet so as to form a hood. When the drape is placed over a flat panel touch screen, the hood slides over the top of the flat panel touch screen to hold the drape in place. The opening at the bottom of the front and the rear sheet facilitates easily sliding the drape on and off the flat panel touch display, and also allows air to circulate from beneath the drape. The flexible layers of the sheets also ease placing and removing the drape.

Surgical drape 10 includes ventilation openings 68 to allow air heated by the flat panel touch screen to escape. Cooled air is often drawn in through the bottom opening. The ventilation openings 68 may be of a suitable shape and number to allow the desired cooling while maintaining the sterile field along display 26. The ventilation openings may be distributed sufficiently evenly across the hood so as to facilitate ventilation of substantially the entire flat panel touch screen, with exemplary embodiments including between about 5 and 50 openings disposed in a single row near top edge 54, with each opening being between 6,4 mm (about 0.25 inches) and 19 mm (about 0.75 inches) in diameter. The openings 68 may be circular, as in the illustrated embodiment, or may have some other shape appropriate for ventilation, for example, rectangular, square, round, oval, and/or generally elongated. In certain embodiments, the openings may have a predetermined relationship with at least some of the vents 38, for example, the same spacing, a similar size, and/or a similar shape to the vents 30.

The drape may be pre-packaged in a sterilized package, and may be used in a single-use manner (with disposal after use for a single patient). This obviates any necessity to clean and sterilize the drape after use.

## Claims

1. A sterile drape (10) for a flat panel display (22) having a display surface (26), comprising:
a sterile front sheet (50) at least as large as the display surface, the front sheet comprising a thin layer of a transparent material and having a top edge (54) and a bottom edge (60); and
a rear sheet (52) , attached to the front sheet near the top edge and having a rear bottom edge (62) detached from the front sheet so as to form a hood with an open bottom, the front sheet and the rear sheet configured for draping over the flat panel display so as to maintain a sterile field adjacent the display surface, with left and right edges of the front and rear sheets being fused together, the sterile drape **characterized in that** the rear sheet has at least one opening (68) for ventilation
**in that**
the rear sheet is formed from a folded extension of the front sheet, and **in that** the rear sheet is significantly shorter than a height of the front sheet between the top edge and the bottom edge so as to leave a lower rear portion of the flat panel display uncovered.

2. The sterile drape of claim 1, wherein the front sheet is substantially rectangular in shape, and wherein the rear sheet has a plurality of openings distributed across the rear sheet near the top edge.

3. The sterile drape of claim 1 or 2, wherein the rear sheet is substantially rectangular in shape.

4. The sterile drape of any one of the preceding claims, wherein the front sheet and the rear sheet comprise a thin layer of flexible polymer material suitable for viewing the display surface therethrough.

5. The sterile drape of claim 4, wherein the thin layer is configured to accommodate the transmission of tactile touch-screen inputs from the sterile field to the flat panel display while maintaining the sterile field.

6. The sterile drape of any one of the preceding claims, wherein the sterile drape has a bottom opening between the front sheet and the rear sheet corresponding to a bottom portion of the flat panel display when the sterile drape is positioned thereon, and wherein the at least one opening comprises a plurality of openings distributed between the left and right edges of the rear sheet sufficiently near the top edge to allow air heated by the flat panel display to enter the bottom opening and exit the plurality of openings such that substantially the entire flat panel display is ventilated and cooled.

7. The sterile drape of any one of the preceding claims, the front sheet and rear sheet having a bottom opening therebetween, the bottom opening for slidably receiving the flat panel display so that the front sheet remains aligned with the display surface when touch commands are transmitted from a user in the sterile field thereto and the bottom remains open;
the at least one ventilation opening in the thin transparent material being near the top edge for ventilation to flow in through the bottom opening and out through the at least one opening during use.

## Patentansprüche

1. Sterile Abdeckung (10) für einen Flachbildschirm (22) mit einer Bildschirmfläche (26), die aufweist:
eine sterile vordere Lage (50), die mindestens so groß ist wie die Bildschirmfläche, wobei die vordere Lage eine dünne Schicht eines transparenten Materials aufweist und eine obere Kante (54) und eine untere Kante (60); und
eine hintere Lage (52), die nahe der oberen Kante an der vorderen Lage angebracht ist und eine von der vorderen Lage getrennte hintere untere Kante (62) aufweist, um eine Haube mit einem offenen Boden auszubilden, wobei die vordere Lage und die hintere Lage eingerichtet sind, über den Flachbildschirm gehängt zu werden, um einen an die Bildschirmfläche angrenzenden sterilen Bereich beizubehalten, der mit linken und rechten Kanten der vorderen und hinteren Lage zusammengeschmolzen ist, wobei die sterile Abdeckung **dadurch gekennzeichnet ist, dass** die hintere Lage mindestens eine Öffnung (68) zur Belüftung aufweist und
dadurch, dass die hintere Lage aus einer gefalteten Erweiterung der vorderen Lage gebildet ist, und
dadurch, dass die hintere Lage signifikant kürzer als eine Höhe der vorderen Lage zwischen der oberen Kante und der unteren Kante ist, um einen unteren hinteren Abschnitt des Flachbildschirms unbedeckt zu lassen.

2. Sterile Abdeckung nach Anspruch 1, bei der die vordere Lage im Wesentlichen rechteckförmig ist und bei der die hintere Lage eine Vielzahl von Öffnungen aufweist, die entlang der hinteren Lage nahe der oberen Kante verteilt sind.

3. Sterile Abdeckung nach Anspruch 1 oder 2, bei der die hintere Lage im Wesentlichen rechteckförmig ist.

4. Sterile Abdeckung nach einem der vorstehenden Ansprüche, bei der die vordere Lage und die hintere Lage eine dünne Schicht eines nachgiebigen Polymermaterials aufweisen, durch das die Bildschirmfläche zu sehen ist.

5. Sterile Abdeckung nach Anspruch 4, bei der die dünne Schicht eingerichtet ist, die Übertragung der taktilen Touchscreeneingaben von dem sterilen Bereich zu dem Flachbildschirm unter Beibehaltung des sterilen Bereichs auszuführen.

6. Sterile Abdeckung nach einem der vorstehenden Ansprüche, die zwischen der vorderen Lage und der hinteren Lage eine Bodenöffnung aufweist, die mit einem Bodenabschnitt des Flachbildschirms übereinstimmt, wenn die sterile Abdeckung darauf angeordnet ist, und wobei die mindestens eine Öffnung eine Vielzahl von Öffnungen aufweist, die ausreichend nahe an der oberen Kante zwischen der linken und rechten Kante der hinteren Lage angeordnet sind, um es durch den Flachbildschirm aufgeheizter Luft zu ermöglichen, in die Bodenöffnung einzutreten und aus der Vielzahl von Öffnungen auszutreten, sodass im Wesentlichen der gesamte Flachbildschirm belüftet und gekühlt wird.

7. Sterile Abdeckung nach einem der vorstehenden Ansprüche, bei der die vordere Lage und hintere Lage zwischen sich eine Bodenöffnung zur verschiebbaren Aufnahme des Flachbildschirms aufweisen, sodass die vordere Lage an der Bildschirmfläche ausgerichtet bleibt, wenn zu dieser Touchbefehle von einem User in dem sterilen Bereich übertragen werden, und der Boden offen bleibt; wobei
die mindestens eine Belüftungsöffnung in dem dünnen transparenten Material zum Belüfteten nahe der oberen Kante ist, um bei Verwendung durch die Bodenöffnung einzuströmen und durch die mindestens eine Öffnung auszuströmen.

## Revendications

1. Champ stérile (10) pour un écran plat (22) ayant une surface d'affichage (26), comprenant :
une plaque frontale stérile (50) au moins aussi grande que la surface d'affichage, la plaque frontale comprenant une fine couche d'un matériau transparent et ayant un bord supérieur (54) et un bord inférieur (60) ; et
une plaque arrière (52) fixée à la plaque frontale près du bord supérieur et ayant un bord inférieur arrière (62) détaché de la plaque frontale de sorte à former un capot avec un fond ouvert, la plaque frontale et la plaque arrière étant configurées pour un drapage sur l'écran plat de sorte à maintenir un champ stérile adjacent à la surface d'affichage, avec des bords gauche et droit des plaques frontale et arrière fusionnés ensemble, le champ stérile étant **caractérisé en ce que** la plaque arrière présente au moins une ouverture (68) pour une ventilation, **en ce que** la plaque arrière est formée d'une extension pliée de la plaque frontale et **en ce que** la plaque arrière est significativement plus courte qu'une hauteur de la plaque frontale entre le bord supérieur et le bord inférieur de sorte à laisser une partie arrière inférieure de l'écran d'affichage non-couverte.

2. Champ stérile de la revendication 1, dans lequel la plaque frontale est essentiellement de forme rectangulaire, et dans lequel la plaque arrière a une pluralité d'ouvertures distribuées à travers la plaque arrière près du bord supérieur.

3. Champ stérile de la revendication 1 ou 2, dans lequel la plaque arrière est essentiellement de forme rectangulaire.

4. Champ stérile de l'une quelconque des revendications précédentes, dans lequel la plaque frontale et la plaque arrière comprennent une fine couche de matériau polymère souple adaptée pour permettre de voir à travers elle la surface d'affichage.

5. Champ stérile de la revendication 4, dans lequel la fine couche est configurée pour recevoir la transmission d'entrées d'écran tactile à partir du champ stérile à l'écran plat tout en maintenant le champ stérile.

6. Champ stérile selon l'une quelconque des revendications précédentes, dans lequel le champ stérile présente une ouverture de fond entre la plaque frontale et la plaque arrière correspondant à une partie inférieure de l'écran plat lorsque le champ stérile est positionné sur celui-ci, et dans lequel l'au moins une ouverture comprend une pluralité d'ouvertures réparties entre les bords gauche et droit de la plaque arrière suffisamment près du bord supérieur afin de permettre à l'air chauffé par l'écran plat de pénétrer dans l'ouverture de fond et de sortir de la pluralité d'ouvertures de sorte que essentiellement l'ensemble de l'écran plat soit ventilé et refroidi.

7. Champ stérile de l'une quelconque des revendications précédentes, la plaque frontale et la plaque arrière ayant une ouverture de fond entre elles, l'ouverture de fond pour recevoir en coulissement l'écran plat de sorte que la plaque frontale reste alignée avec la surface d'affichage lorsque des commandes tactiles sont transmises depuis un utilisateur dans le champ stérile à celui-ci et le fond reste ouvert ;
l'au moins une ouverture de ventilation dans le un matériau transparent fin étant près du bord supérieur pour une ventilation afin d'entrer à travers l'ouverture de fond et de sortir à travers l'au moins une ouverture pendant l'utilisation.
